# FASCICULE DE BREVET EUROPEEN

(11) **EP 1 066 875 B1**
(45) Date de publication et mention de la délivrance du brevet: **06.04.2005**
(21) Numéro de dépôt: 00401334.8
(22) Date de dépôt: 16.05.2000
(51) Int. Cl.: B01F 17/00, A61K 7/00

(54) **Composition sous forme d'émulsion eau-dans-huile ayant une vitesse de cisaillement évolutive**
Wasser in Öl emulsionsförmige Zusammensetzung mit verlaufsveränderlicher Schergeschwindigkeit
Water-in-oil composition with a variable shear rate

(30) Priorité: 06.07.1999 FR 9908712
(43) Date de publication de la demande: 10.01.2001
(73) Titulaire: L'OREAL, 75008 Paris (FR)
(72) Inventeur: Afriat, Isabelle, 75003 Paris (FR); Boulier Wozniak, Virginie, 95520 Osny (FR)
(74) Mandataire: Rasson, Catherine

(56) Documents cités:
- EP-A- 0 612 517
- EP-A- 0 670 157
- EP-A- 0 965 331
- EP-A- 0 970 682
- WO-A-93/14742
- WO-A-95/15812
- WO-A-99/47111

## Description

L'invention se rapporte à une composition se présentant sous forme d'une émulsion eau-dans-huile (E/H) comportant une forte teneur en eau et un tensioactif siliconé particulier. Cette composition a l'aspect d'une crème et est utilisable en particulier dans les domaines cosmétique et/ou dermatologique.

Dans les domaines cosmétique ou dermatologique, il est courant d'utiliser des compositions ayant l'aspect d'une crème et constituées d'une émulsion eau-dans-huile (E/H) comportant une phase aqueuse dispersée dans une phase huileuse. Ces émulsions comportent une phase continue huileuse et permettent donc de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches.

Une crème est, dans les domaines considérés, une composition présentant une certaine viscosité, par opposition aux compositions liquides ou semi-liquides telles que les lotions et les laits, ou encore aux compositions solides.

Toutefois, les crèmes sous forme d'émulsions E/H présentent l'inconvénient d'apporter sur la peau à l'application, un toucher assez gras, la phase huileuse étant la phase externe. Ainsi, ces crèmes sont en général utilisées pour les peaux sèches, étant trop grasses pour être utilisées sur les peaux grasses. De plus, les émulsions E/H n'apportent aucune fraîcheur et sont généralement trop riches en huiles pour être utilisées pendant l'été ou dans les pays chauds.

Pour surmonter ces inconvénients, il a été envisagé de préparer des émulsions à forte teneur en eau. Toutefois, la teneur en eau ne peut pas être trop importante pour des raisons de stabilité, ou alors une forte teneur en eau doit être compensée par l'ajout de plusieurs tensioactifs ou d'agents gélifiants qui peuvent nuire au confort de la composition finale et même entraîner des problèmes d'irritations cutanés notamment chez les sujets à peaux sensibles.

Il subsiste donc le besoin d'une composition ayant la viscosité d'une crème et se présentant sous forme d'une émulsion eau-dans-huile stable, comportant une quantité importante d'eau et utilisable dans les domaines cosmétique et/ou dermatologique, qui ne présente pas les inconvénients de l'art antérieur.

La demanderesse a maintenant trouvé une composition du type émulsion eau dans huile permettant d'atteindre ces objectifs.

L'invention a pour objet une composition comprenant, dans un milieu physiologiquement acceptable, une phase aqueuse dispersée dans une phase huileuse à l'aide d'un agent émulsionnant siliconé, caractérisée par le fait que la phase aqueuse représente au moins 80 % en poids par rapport au poids total de la composition, que l'agent émulsionnant est un alkyldiméthicone copolyol ayant un HLB inférieur à 8 choisi parmi l'oleyldiméthicone copoylol, le stéaryldiméthicone copolyol et leurs mélanges, et que le rapport pondéral phase huileuse/agent émulsionnant est égal ou supérieur à 5.

On entend par « milieu physiologiquement acceptable » un milieu compatible avec la peau, les lèvres, les ongles, le cuir chevelu et/ou les cheveux.

En dépit de la quantité importante d'eau, la composition de l'invention est stable dans le temps. En outre, elle possède une caractéristique rhéologique spécifique qui rend son utilisation dans les domaines considérés, particulièrement intéressante. En effet, lors de l'application sur la peau, elle "casse", c'est-à-dire qu'elle se fluidifie brutalement sous l'effet du cisaillement, ce qui est probablement dû à un phénomène de rupture au sein de l'émulsion. Ainsi, la composition de l'invention apporte une très grande fraîcheur sur la peau.

La composition obtenue selon l'invention présente une viscosité allant de 1,9 Pa.s (19 poises) à 20 Pa.s (200 poises). Cette viscosité est mesurée au Rhéomat 180, c'est-à-dire avec l'appareil RM180 Rheomat de la société METTLER (mobile 4), à un taux de cisaillement de 200 s⁻¹ et à 25 °C.

La composition selon l'invention comporte au moins 80 % en poids de phase aqueuse par rapport au poids total de la composition et de préférence au moins 82 % du poids total de la composition. La phase aqueuse peut constituer jusqu'à 92 % du poids total de la composition. L'eau constitue avantageusement au moins 70 % et de préférence au moins 75 % du poids total de la composition.

Par ailleurs, la phase aqueuse de l'émulsion peut contenir un ou plusieurs alcools inférieurs tels que l'éthanol, en une quantité allant de préférence jusqu'à 15 % et mieux jusqu'à 10 % du poids total de la composition, un ou plusieurs polyols tels que la glycérine et le propylène glycol en une quantité allant par exemple jusqu'à 20 % et mieux jusqu'à 10 % du poids total de la composition.

L'agent émulsionnant utilisé dans la composition de l'invention est un alkyldiméthicone copolyol ou un mélange d'alkyldiméthicone copolyols choisi parmi l'oleyldiméthicone copoylol, le stéaryldiméthicone copolyol et leurs mélanges qui constitue de préférence le seul type d'agent émulsionnant. Cet agent émulsionnant a un HLB (Hydrophilic Lipophilic balance) inférieur à 8 et de préférence inférieur à 6. Comme alkyldiméthicone copolyol, on peut utiliser par exemple l'oleyldiméthicone copolyol comme celui vendu sous la dénomination KF-6026 par la Société Shin-Etsu, le stéaryldiméthicone copolyol comme celui vendu sous la dénomination X-22-904 par la Société Shin-Etsu.

L'agent émulsionnant siliconé est présent en une quantité en matière active allant de préférence de 0,5 à 4 % et mieux de 0,8 à 4 % en poids par rapport au poids total de la composition.

Même quand la composition est exempte de tout autre agent émulsionnant, elle présente une excellente stabilité dans le temps.

Le rapport pondéral phase huileuse/agent émulsionnant est égal ou supérieur à 5 et de préférence égal ou supérieur à 8.

La phase huileuse de la composition selon l'invention peut renfermer toute sorte d'huiles et de corps gras bien connus de l'homme du métier, comme exemple les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkyl-benzoates), les huiles de silicone volatiles ou non volatiles telles que les polydiméthylsiloxanes (PDMS) et les cyclodiméthylsiloxanes ou cyclométhicones, et les huiles fluorées ou fluorosiliconées, ainsi que les mélanges de ces huiles.

De préférence, la phase huileuse de la composition de l'invention comprend au moins une huile de silicone volatile généralement présente en une quantité d'au moins 5 % en poids et de préférence allant de 5 à 25 % en poids par rapport au poids total de la composition. Comme huile de silicone volatile, on peut citer par exemple les silicones cycliques (ou cyclométhicones) telles que la pentacyclométhicone, la tétracyclométhicone ou l'hexacyclométhicone.

La phase huileuse peut contenir, en outre, d'autres constituants gras tels que les alcools gras comme l'alcool stéarylique, l'alcool cétylique et l'alcool cétéarylique, et les acides gras.

La phase huileuse est présente dans la composition selon l'invention en une quantité allant généralement de 7,5 à 20 % et de préférence de 10 à 18 % en poids par rapport au poids total de la composition.

Un autre avantage de la composition selon l'invention provient de ce qu'on peut y incorporer une grande quantité d'électrolyte sans nuire à la stabilité de la composition.

Comme électrolyte, on peut citer par exemple les sels des métaux mono-, di- ou trivalents, et plus particulièrement les sels de métal alcalino-terreux tels que les sels de baryum, de calcium et de strontium ; les sels de métal alcalin tels que les sels de sodium et de potassium, les sels de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

Les ions constituant ces sels peuvent être choisis par exemple parmi les carbonates, les bicarbonates, les sulfates, les glycérophosphates, les borates, les chlorures, les bromures, les nitrates, les acétates, les hydroxydes, les persulfates ainsi que les sels d'α-hydroxyacides (citrates, tartrates, lactates, malates) ou d'acides de fruits, ou encore les sels d'acides aminés (aspartate, arginate, glycocholate, fumarate).

De préférence, l'électrolyte est un mélange de sels comprenant notamment des sels de calcium, de magnésium, et de sodium, et notamment un mélange comprenant au moins du chlorure de magnésium, du chlorure de potassium, du chlorure de sodium, du chlorure de calcium, du bromure de magnésium, le dit mélange correspondant à des sels de la mer morte.

La teneur en électrolyte, lorsque la composition en contient, va en général de 0,5 à 20 % et de préférence de 2,5 à 10 % en poids par rapport au poids total de la composition.

La composition selon l'invention trouve son application dans un grand nombre de traitements notamment cosmétiques de la peau, y compris du cuir chevelu, des cheveux, des ongles, et/ou des muqueuses. en particulier pour le soin, le nettoyage, le maquillage et/ou la protection solaire de la peau et/ou des muqueuses, ainsi que pour la préparation d'une crème destinée au traitement de la peau, plus particulièrement de la peau grasse (apport de fraîcheur).

Aussi, la présente invention a pour objet l'utilisation cosmétique de la composition telle que définie ci-dessus, pour le traitement, la protection, le soin, le démaquillage et/ou le nettoyage de la peau, des lèvres et/ou des cheveux, et/ou pour le maquillage de la peau et/ou des lèvres.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition telle que définie ci-dessus.

L'invention a aussi pour objet l'utilisation de la composition telle que définie ci-dessus pour la fabrication d'une crème destinée au traitement des peaux grasses.

De façon connue, la composition de l'invention peut contenir également des adjuvants habituels dans les domaines cosmétique et/ou dermatologique, tels que les actifs, les conservateurs, les antioxydants, les agents complexants, les solvants, les parfums, les charges, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme actifs, on peut citer notamment, outre les électrolytes indiqués ci-dessus, les hydratants et par exemple les hydrolysats de protéines et les polyols tels que la glycérine, les glycols comme les polyéthylène glycols, et les dérivés de sucre ; les extraits naturels ; les oligomères procyannidoliques ; les vitamines ; l'urée ; les dépigmentants tels que l'acide kojique et l'acide caféique ; les bêta-hydroxyacides tels que l'acide salicylique et ses dérivés ; les alpha-hydroxyacides tels que l'acide lactique et l'acide glycolique ; les rétinoïdes tels que le rétinol et les caroténoïdes ; les filtres, et leurs mélanges.

Le ou les actifs peuvent être par exemple présents en une concentration allant de 0,01 à 20 %, de préférence de 0,1 à 5 % et mieux de 0,5 à 3 % du poids total de la composition.

Les exemples ci-après de compositions selon l'invention sont donnés à titre d'illustration et sans caractère limitatif. Les quantités y sont données en % en poids, sauf mention contraire.

### Exemple 1: Base de maquillage

### A. Phase huileuse

- Oléyldiméthicone copolyol 1 %
- Pentacyclométhicone 5,75 %
- Mélange de cétéaryl octanoate et de myristate d'isopropyle 5,75 %

### B. Phase aqueuse

- Chlorure de sodium 2,5 %
- Eau 85 %

Mode opératoire : on prépare séparément les phases aqueuse et huileuse, puis on introduit peu à peu sous agitation modérée la phase aqueuse dans la phase huileuse.

On obtient une crème blanche ayant une viscosité mesurée à température ambiante (environ 20-25°C) au RHEOMAT 180, de 6,38 Pa.s (63,8 poises) au temps zéro. Cette viscosité se stabilise après 10 minutes à 4,99 Pa.s (49,9 poises).

La crème obtenue est apte à hydrater et assouplir la peau.

### Exemple 2: crème après solaire pour le corps

### A. Phase huileuse

- Sféaryldiméthicone copolyol 2 %
- Pentacyclométhicone 18 %

### B. Phase aqueuse

- Chlorure de sodium 2,5 %
- Eau 77,5 %

Mode opératoire : on prépare séparément les phases aqueuse et huileuse, puis on introduit peu à peu sous agitation modérée la phase aqueuse dans la phase huileuse.

On obtient une crème blanche ayant une viscosité mesurée à température ambiante (environ 20-25°C) au RHEOMAT 180, de 2,36 Pa.s (23,6 poises) au temps zéro. Cette viscosité se stabilise après 10 minutes à 2,02 Pa.s (20,2 poises).

La crème obtenue est apte à rafraîchir la peau et lui procurer un effet satiné.

## Revendications

1. Composition comprenant, dans un milieu physiologiquement acceptable, une phase aqueuse dispersée dans une phase huileuse à l'aide d'un agent émulsionnant siliconé, **caractérisée par le fait que** la phase aqueuse représente au moins 80 % en poids par rapport au poids total de la composition, que l'agent émulsionnant est un alkyldiméthicone copolyol ayant un HLB inférieur à 8 choisi parmi l'oleyldiméthicone copoylol, le stéaryldiméthicone copolyol et leurs mélanges, et que le rapport pondéral phase huileuse/agent émulsionnant est égal ou supérieur à 5.

2. Composition selon la revendication 1, **caractérisée en ce qu'**elle a une viscosité allant de 1,9 Pa.s à 20 Pa.s.

3. Composition selon la revendication 1 ou 2, **caractérisée en ce qu'**elle comporte au moins 70 % d'eau par rapport au poids total de la composition.

4. Composition selon l'une quelconque des revendications précédentes, **caractérisée par le fait que** l'agent émulsionnant est présent en une quantité allant de 0,5 à 6 % en poids par rapport au poids total de la composition.

5. Composition selon l'une quelconque des revendications précédentes, **caractérisée par le fait que** la phase huileuse est présente en une quantité allant de 7,5 à 20 % en poids par rapport au poids total de la composition.

6. Composition selon l'une quelconque des revendications précédentes, **caractérisée par le fait que** le rapport pondéral phase huileuse/agent émulsionnant est égal ou supérieur à 8.

7. Composition selon l'une quelconque des revendications précédentes, **caractérisée par le fait que** la phase huileuse contient au moins une huile de silicone volatile.

8. Composition selon l'une quelconque des revendications précédentes, **caractérisée par le fait qu'**elle contient au moins un électrolyte.

9. Composition selon la revendication précédente, **caractérisée par le fait que** l'électrolyte est présent en une quantité allant de 0,5 à 20 % du poids total de la composition.

10. Composition selon l'une quelconque des revendications précédentes, **caractérisée par le fait qu'**elle contient au moins un actif choisi parmi les hydratants, les extraits naturels, les oligomères procyannidoliques, les vitamines, l'urée, les dépigmentants, les bêta-hydroxyacides, les alpha-hydroxyacides, les rétinoïdes, les filtres, et leurs mélanges.

11. Composition selon l'une quelconque des revendications précédentes, **caractérisée en ce qu'**elle constitue une composition cosmétique.

12. Utilisation cosmétique de la composition selon l'une quelconque des revendications 1 à 11, pour le traitement, la protection, le soin, le démaquillage et/ou le nettoyage de la peau, des lèvres et/ou des cheveux, et/ou pour le maquillage de la peau et/ou des lèvres.

13. Procédé de traitement cosmétique de la peau, y compris du cuir chevelu, des cheveux, et/ou des lèvres, **caractérisé par le fait que** l'on applique sur la peau, les cheveux et/ou les lèvres, une composition selon l'une quelconque des revendications 1 à 11.

14. Utilisation de la composition selon l'une quelconque des revendications 1 à 11 pour la fabrication d'une crème destinée au traitement des peaux grasses.

## Patentansprüche

1. Zusammensetzung, die in einem physiologisch akzeptablen Medium eine mit Hilfe eines Siliconemulgators in einer Ölphase dispergierte wässrige Phase enthält, **dadurch gekennzeichnet, dass** die wässrige Phase, bezogen auf das Gesamtgewicht der Zusammensetzung, mindestens 80 Gew.-% ausmacht, der Emulgätor ein Alkyldimethiconcopolyol mit einem HLB-Wert unter 8 ist, das unter Oleyldimethiconcopolyol, Stearyldimethiconcopolyol und deren Gemischen ausgewählt ist, und das Gewichtsverhältnis Ölphase/Emulgator mindestens 5 beträgt.

2. Zusammensetzung nach Anspruch 1, **dadurch gekennzeichnet, dass** sie eine Viskosität von 1,9 bis 20 Pa·s aufweist.

3. Zusammensetzung nach Anspruch 1 oder 2, **dadurch,gekennzeichnet, dass** sie, bezogen auf das Gesamtgewicht der Zusammensetzung, mindestens 70 % Wasser enthält.

4. Zusammensetzung nach einem der vorhergehenden Ansprüche, **dadurch gekennzeichnet, dass** der Emulgator in einem Mengenanteil von 0,5 bis 6 Gew.-%, bezogen auf das Gesamtgewicht der Zusammensetzung, enthalten ist.

5. Zusammensetzung nach einem der vorhergehenden Ansprüche, **dadurch gekennzeichnet, dass** die Ölphase in einer Menge von 7,5 bis 20 Gew.-%, bezogen auf das Gesamtgewicht der Zusammensetzung, vorliegt.

6. Zusammensetzung nach einem der vorhergehenden Ansprüche, **dadurch gekennzeichnet, dass** das Gewichtsverhältnis Ölphase/Emulgator mindestens 8 beträgt.

7. Zusammensetzung nach einem der vorhergehenden Ansprüche, **dadurch gekennzeichnet, dass** die Ölphase mindestens ein flüchtiges Siliconöl enthält.

8. Zusammensetzung nach einem der vorhergehenden Ansprüche, **dadurch gekennzeichnet, dass** sie mindestens einen Elektrolyten enthält.

9. Zusammensetzung nach dem vorhergehenden Anspruch, **dadurch gekennzeichnet, dass** der Elektrolyt in einer Menge von 0,5 bis 20 % des Gesamtgewichts der Zusammensetzung enthalten ist.

10. Zusammensetzung nach einem der vorhergehenden Ansprüche, **dadurch gekennzeichnet, dass** sie mindestens einen Wirkstoff enthält, der unter den Hydratisierungsmitteln, natürlichen Extrakten, oligomeren Procyanidinen, Vitaminen, Harnstoff, Depigmentierungsmitteln, β-Hydroxysauren, α-Hydroxysäuren, Retinoiden, Filtern und deren Gemischen ausgewählt ist.

11. Zusammensetzung nach einem der vorhergehenden Ansprüche, **dadurch gekennzeichnet, dass** sie eine kosmetische Zusammensetzung ist.

12. Kosmetische Verwendung der Zusammensetzung nach einem der Ansprüche 1 bis 17 zur Behandlung, zum Schutz, zur Pflege, zum Abschminken und/oder zur Reinigung der Haut, der Lippen und/oder der Haare und/oder zum Schminken der Haut und/ oder der Lippen.

13. Verfahren zur kosmetischen Behandlung der Haut einschließlich der Kopfhaut, der Haare und/oder der Lippen, **dadurch gekennzeichnet, dass** auf die Haut, die Haare und/oder die Lippen eine Zusammensetzung nach einem der Ansprüche 1 bis 11 aufgetragen wird.

14. Verwendung der Zusammensetzung nach einem der Ansprüche 1 bis 11 zur Herstellung einer Creme, die zur Behandlung von fettiger Haut vorgesehen ist.

## Claims

1. Composition comprising, in a physiologically acceptable medium, an aqueous phase dispersed in an oily phase with the aid of a silicone emulsifier, **characterized in that** the aqueous phase represents at least 80% by weight relative to the total weight of the composition, **in that** the emulsifier is an alkyldimethicone copolyol having an HLB of less than 8 chosen from oleyldimethicone copolyol and stearyldimethicone copolyol, and mixtures thereof, and **in that** the oily phase/emulsifier weight ratio is greater than or equal to 5.

2. Composition according to Claim 1, **characterized in that** it has a viscosity ranging from 1.9 Pa.s to 20 Pa.s.

3. Composition according to Claim 1 or 2, **characterized in that** it comprises at least 70% water relative to the total weight of the composition.

4. Composition according to any one of the preceding claims, **characterized in that** the emulsifier is present in an amount ranging from 0.5 to 6% by weight relative to the total weight of the composition.

5. Composition according to any one of the preceding claims, **characterized in that** the oily phase is present in an amount ranging from 7.5 to 20% by weight relative to the total weight of the composition.

6. Composition according to any one of the preceding claims, **characterized in that** the oily phase/emulsifier weight ratio is greater than or equal to 8.

7. Composition according to any one of the preceding claims, **characterized in that** the oily phase contains at least one volatile silicone oil.

8. Composition according to any one of the preceding claims, **characterized in that** it contains at least one electrolyte.

9. Composition according to the preceding claim, **characterized in that** the electrolyte is present in an amount ranging from 0.5 to 20% relative to the total weight of the composition.

10. Composition according to any one of the preceding claims, **characterized in that** it contains at least one active agent chosen from moisturizers, natural extracts, procyanidol oligomers, vitamins, urea, depigmenting agents, beta-hydroxy acids, alpha-hydroxy acids, retinoids and screening agents, and mixtures thereof.

11. Composition according to any one of the preceding claims, **characterized in that** it constitutes a cosmetic composition.

12. Cosmetic use of the composition according to any one of Claims 1 to 11, to treat, protect, care for, remove make-up from and/or cleanse the skin, the lips and/or the hair, and/or to make up the skin and/or the lips.

13. Cosmetic treatment process for the skin, including the scalp, the hair and/or the lips, **characterized in that** a composition according to any one of Claims 1 to 11 is applied to the skin, the hair and/or the lips.

14. Use of the composition according to any one of Claims 1 to 11 to manufacture a cream intended for treating greasy skin.
